# EUROPÄISCHE PATENTSCHRIFT

(11) **EP 2 982 974 B1**
(45) Veröffentlichungstag und Bekanntmachung des Hinweises auf die Patenterteilung: **26.10.2016**
(21) Anmeldenummer: 14002749.1
(22) Anmeldetag: 06.08.2014
(51) Int. Cl.: G01N 31/22, G01N 33/28, G01N 21/29

(54) **Verfahren zur Feststellung des Eisengehalts in einem Schmieröl**
Method of determining the iron content in a lubricating oil
Procédé de détermination de la teneur en fer d'une huile de lubrification

(43) Veröffentlichungstag der Anmeldung: 10.02.2016
(73) Patentinhaber: Martechnic GmbH, 22459 Hamburg (DE)
(72) Erfinder: Lagner, Stefan, D-19071 Grambow (DE)
(74) Vertreter: Stoppkotte, Cornelia

(56) Entgegenhaltungen:
- CN-A- 102 718 209
- DE-C1- 19 640 500
- US-A- 3 669 631
- US-A- 4 238 197
- SALVADOR A ET AL: "Determination of the total iron content of used lubricating oils by atomic-absorption with use of emulsions", TALANTA, ELSEVIER, AMSTERDAM, NL, vol. 30, no. 12, 1 December 1983 (1983-12-01), pages 986-988, XP026596345, ISSN: 0039-9140, DOI: 10.1016/0039-9140(83)80230-6 [retrieved on 1983-12-01]

## Beschreibung

Die Erfindung betrifft ein Verfahren zur Feststellung des Eisengehalts in einem Schmieröl einer Schiffsantriebsmaschine.

Heutige Schiffsmaschinen in Containerschiffen, Tankschiffen, Fähren oder Kreuzfahrtschiffen verwenden üblicherweise langsam laufende Zweitaktmotoren mit mehr als 5 Zylindern, von denen eine hohe Lebensdauer erwartet wird. Die Größe derartiger Maschinen bedingt ein großes Schmierölvolumen.

Die Schmierung zwischen Kolbenringen und Laufbuchsen erfolgt dabei in der Regel als Verlustschmierung, da der hier entstehende metallische Abrieb erheblich größer ist als im Bereich des Kurbeltriebs. Zur Verringerung des Schmierölverbrauchs und gleichzeitig des Erhalts einer ausreichenden Schmierung ist die Überwachung des verwendeten Schmieröls von besonderer Bedeutung. Insbesondere ist der Anteil von Eisenpartikeln in dem Schmieröl zu überwachen, da ein hoher Eisengehalt darauf hindeutet, dass es Probleme im Zusammenspiel zwischen Kolbenringen, Kolben und Laufbuchse gibt.

Der Eisengehalt in einem Schmieröl ergibt sich einerseits aus mechanischem Abrieb in Form von Eisenpartikeln, d.h. elementarem Eisen, und anderseits durch Säurekorrosion, welche zu Eisensalzen, d.h. gelöstem oder korrodiertem Eisen führt, insbesondere bei hohem Schwefelgehalt des verwendeten Kraftstoffs. Hierbei ist auch von Bedeutung, dass heutige Schiffe, insbesondere Containerschiffe, häufig nur mit einer Teilbeladung gefahren werden oder zur Verringerung des Kraftstoffverbrauchs mit reduzierter Geschwindigkeit gefahren werden. Beides führt dazu, dass die Schiffsmaschine nicht mit optimaler Leistung betrieben wird, sondern unter Teillast, was zu niedrigeren Betriebstemperaturen der Schiffsmaschine und damit zu erhöhter Korrosion führt.

Der Eisengehalt eines Schmieröls kann unter Laborbedingungen zwar relativ genau bestimmt werden. Derartige Laborbedingungen stehen jedoch im laufenden Schiffsbetrieb nicht zur Verfügung. Eine hochgenaue Feststellung des Eisengehalts in einem Schmieröl ist jedoch im laufenden Betrieb auch nicht erforderlich, da es ausreicht, während des Betriebs einen stabilen Zustand des Schmieröls festzustellen oder frühzeitig Tendenzen erkennen zu können, die eine ungewöhnliche Verschlechterung des Schmieröls bedeuten können.

US 4 238 197 offenbart ein Verfahren zur Feststellung des Eisengehalts in Schmierölen, wobei eine Probe des Schmieröls mit einer Schwefelsäure, Isoamylalkohol, Natriumazetat, Hydroxylamin Hydrochlorid und Kalisalz enhältenden Reagenzlösung gemischt wird. Eine Phasentrennung und ein Farbvergleich folgen.

Der Erfindung liegt daher die Aufgabe zugrunde, ein Verfahren zur Feststellung des Eisengehalts in einem Schmieröl einer Schiffsantriebsmaschine anzugeben, mit dessen Hilfe die Nutzqualität des Schmieröls und der Verschleißzustand einer Maschine auf einfache Weise im laufenden Betriebszustand der Maschine ermittelt werden kann.

Diese Aufgabe wird durch die Im Anspruch 1 angegebenen Merkmale der Erfindung gelöst. Vorteilhafte Weiterbildungen der Erfindung sind in Unteransprüchen angegeben.

Das erfindungsgemäße Verfahren zur Feststellung des Eisengehalts in einem Schmieröl einer Schiffsantriebsmaschine ist insbesondere durch die folgenden Schritte gekennzeichnet: es wird eine Reagenzlösung mit Kaliumhexacyanoferrat (II) (auch gelbes Blutlaugensalz genannt) bereitgestellt, auf deren Oberfläche eine Probe des Schmieröls zur Bildung einer Messprobe aufgebracht wird. Die Messprobe wird für eine Zeitdauer zwischen 4 und 20 Minuten und bei einer erhöhten Temperatur zwischen 40 und 80°C, vorzugsweise 60°C, ruhig gehalten. Dann wird ein Farbvergleich zwischen der Färbung der Reagenzlösung mit einer Farbskala durchgeführt, wobei der Grad der Färbung ein Maß für den Eisengehalt des Schmieröls darstellt.

Zur Messung des Gesamteisengehalts umfasst die Reagenzlösung dabei vorzugsweise Salpetersäure in einer Konzentration von 0,1 bis 0,5 % und Kaliumhexacyanoferrat (II) in einer Konzentration von 0,002 bis 0,05% in gereinigtem Wasser.

Zur Herstellung der Reagenzlösung wird vorzugsweise in Pulverform vorliegendes Kaliumhexacyanoferrat (II) Trihydrat mit gereinigtem Wasser zu einer 2%igen Lösung aufbereitet.

Bei den Prozentangaben handelt es sich hierbei um Gewichtsprozente, wobei sich die Gewichtsprozente in Bezug auf Kaliumhexacyanoferrat (II) auf das Trihydrat beziehen.

Zur Ermittlung des Gesamteisengehalts wird besonders bevorzugt eine Reagenzlösung aus 10 ml 0,25%iger Salpetersäure in gereinigtem Wasser und 0,2 ml (ca. 5 Tropfen) einer 2%igen Kaliumhexacyanoferrat (II)-Lösung in gereinigtem Wasser verwendet, womit sich eine Konzentration von 0,04% Kaliumhexacyanoferrat (II) in der Reagenzlösung ergibt.

Zur Ermittlung des korrosiven bzw. gelösten Eisens wird vorzugsweise eine Reagenzlösung verwendet, die gereinigtes Wasser und Kaliumhexacyanoferrat (II) in einer Konzentration von ebenfalls 0,002 bis 0,05% umfasst.

Besonders bevorzugt wird auch hierzu eine Reagenzlösung aus 10 ml gereinigtem Wasser und 0,2 ml einer 2%igen Kaliumhexacyanoferrat (II)-Lösung in gereinigtem Wasser verwendet.

Das erfindungsgemäße Verfahren kann sehr einfach ausgeführt werden, da als Reagenzlösung lediglich eine Mischung aus verdünnter Salpetersäure bzw. gereinigtem Wasser und Kaliumhexacyanoferrat (II) erforderlich ist. Solche Reagenzlösungen sind unkritisch und erfordern keine besonderen Sicherheitsmaßnahmen. Zwar sind bei der Anwendung die einschlägigen Schutzvorschriften zu beachten, aber die Lösungen müssen nicht als Gefahrgut behandelt werden, so dass insbesondere die Lagerung und der Transport der Reagenzlösung sehr kostengünstig gestaltet werden kann.

Der zu messende Wertebereich korrosiven Eisens in einem Öl liegt bei > 10µg/l - 250µg/l, während der zu messende Gesamteisengehalt im Bereich von > 50 mg/l - 1000 mg/l liegt.

Vorzugsweise wird zur Messung des Gesamteisengehalts einer Probe eine Menge von 0,5 ml Schmieröl bei einer Reagenzmenge von ca. 10 ml auf die Oberfläche der mit Salpetersäure und Kaliumhexacyanoferrat (II) erstellten Reagenzlösung aufgebracht und für 8 Minuten ruhig gehalten. Wenn das korrosive Eisen in einer Probe erfasst werden soll, wird die Menge Schmieröls vorzugsweise auf 1 ml bei einer mit Wasser und Kaliumhexacyanoferrat (II) erstellten Reagenzlösung von ca. 10 ml und einer Wartezeit von 15 Minuten erhöht.

Gemäß der Erfindung erfolgt keine Vermischung der Reagenzlösung mit der Ölprobe, sondern die Ölprobe wird lediglich vorsichtig auf die Oberfläche der Reagenzlösung aufgebracht. An der Grenzfläche zwischen Öl und Reagenzlösung bilden sich durch die Wirkung der Salpetersäure aus den Eisenpartikeln Eisenionen (Fe3+), die langsam durch die Reagenzlösung nach unten wandern und darin durch die Reaktion mit Kaliumhexacyanoferrat (II) zu einer Blaufärbung führen. Weiterhin wandern bzw. diffundieren auch in dem Öl bereits enthaltene Eisenionen in die Reagenzlösung. Unter feststehenden Bedingungen von Wartezeit und Temperatur lässt sich damit eine definierte Aussage treffen, wie hoch der Eisengehalt in einer Probe des Schmieröls ist. Der Vergleich der gefärbten Reaktionslösung erfolgt vorzugsweise mittels einer Farbskala mit einer Reihe von farbigen Sichtflächen oder einzelnen Farbkarten, die jeweils für einen bestimmten Eisengehalt in der Probe stehen. Wenn sich aus dem Vergleich zwischen der Reagenzlösung und der Farbskala eine große Abweichung von dem erwarteten Wert ergibt, sind Maßnahmen einzuleiten, um entweder das Öl wieder aufzufrischen, z. B. durch Änderung der Base Number (BN), oder Untersuchungen an der Maschine auszuführen, um festzustellen, welche Ursache einer erhöhten Eisenkonzentration im Schmieröl zugrunde liegt.

Zur Durchführung des erfindungsgemäßen Verfahrens wird dem Anwender vorzugsweise ein Testkit zur Verfügung gestellt, das in einem Transportkoffer aufgenommen ist, welcher ein auf einer geregelte Temperatur von 40° - 80°C regelbaren Heizbehälter zur Aufheizung eines oder mehrerer Messbehälter in einem Wasserbad, eine Vorratsflasche einer wässrigen Salpetersäure, vorzugsweise einer 0,25%igen wässrigen Salpetersäure, eine Vorratsflasche Kaliumhexacyanoferrat (II), vorzugsweise als Kaliumhexacyanoferrat (II) Trihydrat in Pulverform, eine Vorratsflasche gereinigten Wassers, eine Reihe von Messbehältern, vorzugsweise mit einem Volumen von 17 ml, und eine Anzahl von Farbmusterkarten enthält. Der Anwender des Verfahrens hat lediglich den Wasserheizbehälter auf eine bestimmte Temperatur, vorzugsweise 60°C, aufzuheizen und dann eine Reagenzlösung aus der vorhandenen 0,25%igen Salpetersäure oder gereinigten Wassers und Kaliumhexacyanoferrat (II) zu bilden, welche in den Messbehälter eingefüllt wird und etwas geschüttelt wird, um eine gut durchmischte Reagenzlösung zu erhalten. Der Messbehälter wird nun in den Heizbehälter eingestellt, und nach kurzer Zeit hat die Reagenzlösung in dem Messbehälter die gewünschte Temperatur erreicht. Nun kann eine Probe von 0,5 ml bzw. 1 ml des zu untersuchenden Schmieröls in Tropfenform auf die Oberfläche der Reagenzlösung aufgebracht werden. Hierbei soll es keine Vermischung mit der Reagenzlösung geben. Alternativ wird die Probe außerhalb des Heizbehälters auf die Reagenzlösung gegeben und der Messbehälter wird erst dann in das Heizbad eingestellt. Es ist dann eine Wartezeit von vorzugsweise 8 bzw. 15 Minuten einzuhalten, nach der der Messbehälter aus dem Heizbehälter entnommen wird und vor die Farbskala gehalten wird. Das beim Vergleich mit der Farbskala am besten zur Deckung kommende Farbmuster der Reagenzlösung wird nun als dasjenige betrachtet, das dem der Farbskala entsprechenden Wert des Gehalts an Eisen in der Probe entspricht.

Da eine Schiffsmaschine über mehrere Zylinder verfügt, können mehrere Proben gleichseitig erfasst und ausgewertet werden.

Die Erfindung wird nachstehend anhand eines Ausführungsbeispiels näher erläutert. Es zeigen:
- Fig, 1: eine tabellarische Darstellung des Verlaufs der Chrominanz/Luminanz einer Reagenzlösung in Abhängigkeit vom Gesamteisengehalt, und

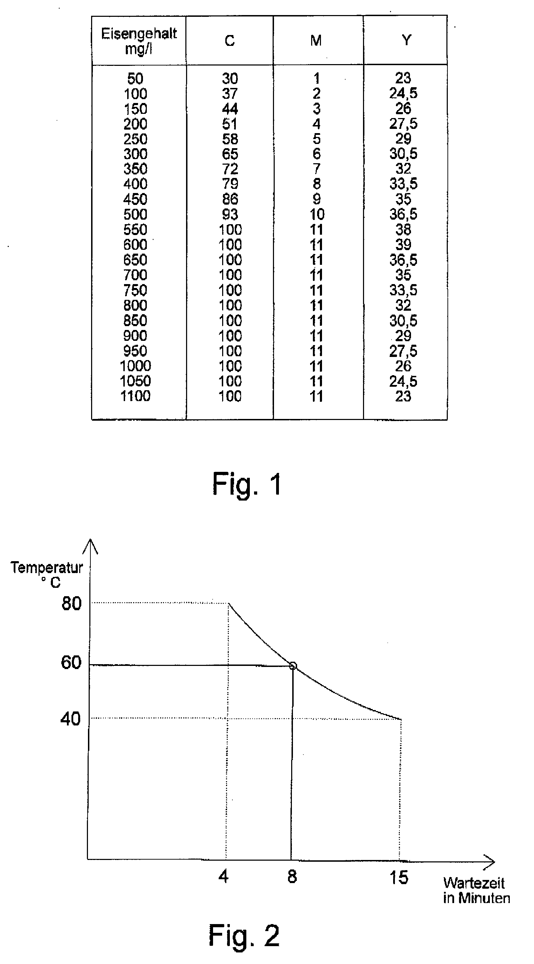

- Fig. 2: eine grafische Darstellung eines Zeit-/Temperaturverlaufs.

Bei dem erfindungsgemäßen Verfahren zur Feststellung des Eisengehalts in einem Schmieröl einer Schiffsantriebsmaschine kann sowohl der Gesamteisengehalt als auch allein das korrosive Eisen in dem Öl ermittelt werden. Korrosives Eisen bildet sich in einem Schmieröl insbesondere aufgrund eines hohen Schwefelgehalts in Verbindung mit niedrigen Betriebstemperaturen der Maschine.

Mit dem erfindungsgemäßen Verfahren kann korrosives Eisen ab einer Menge von etwa 10 µg/l im Schmieröl festgestellt werden.

Der Gesamteisengehalt in einem Öl wird insbesondere durch Abriebeisen bestimmt, so dass der Gesamteisengehalt in einem Öl ab einem Gehalt von 50 mg/l relevant wird. Zur Durchführung einer Messung des Gesamteisengehalts sieht die Erfindung vor, eine Reagenzlösung mit einer 0,25%igen Salpetersäure in gereinigtem Wasser bereit zu halten, der Kaliumhexacyanoferrat (II) Trihydrat als 2%ige wässrige Lösung zugefügt wird. Es wird dazu 10 ml der angegebenen Salpetersäure mit 0,2 ml Kaliumhexacyanoferrat (II) Lösung vermischt. Die Vermischung erfolgt in einem Messbehälter mit einem Innendurchmesser von 22,5 mm durch mehrfaches Schütteln. Nun wird die Reagenzlösung in ein auf 60°C erhitztes Wasserbad eingestellt und nach kurzer Zeit hat die Reagenzlösung selbst eine durchgehende Temperatur von 60°C. erreicht. Dadurch ergibt sich ein definierter Ausgangszustand. Nun wird eine Probe Schmieröl von 0,5 ml, z. B. mittels einer Pipette, auf die Oberfläche der Reagenzlösung gegeben. Das Schmieröl verteilt sich auf der Oberfläche der Reagenzlösung. An der Grenzfläche zwischen Reagenzlösung und Schmieröl treten nun Eisenpartikel in die Reagenzlösung ein und es bilden sich Fe3+-Ionen, die langsam nach unten durch die Reagenzlösung wandern und darin zu einer Verfärbung der Reagenzlösung in Abhängigkeit von der Menge der im Schmieröl enthaltenen Eisenteilchen führen. Nach einer definierten Zeitspanne von 8 Minuten wird der Messwertbehälter aus dem Wasserbad entnommen und unmittelbar vor eine Farbskala oder verschiedene Farbkarten bestimmter Chrominanz und Luminanz gestellt. Es wird nun ein visueller Vergleich der Färbung in der Reagenzlösung mit der Farbe der Farbskala oder den Farbkarten durchgeführt. Die Chrominanz einer Farbkarte, die der Färbung in der Reagenzlösung entspricht, zeigt den Gesamteisengehalt in der gemessenen Probe unter festgelegten Bedingungen, wie Temperatur, Zeitdauer und Größe der Kontaktoberfläche im Messbehälter.

Es hat sich gezeigt, dass eine Wartedauer von 8 Minuten bei einer Temperatur von 60°C und den angegebenen Mengenverhältnissen sowie der Größe der Kontaktfläche im Messgefäß gut zu handhabende Bedingungen für ein standardisiertes Messverfahren darstellen.

Wenn die Temperatur geringer gewählt ist, ist die Wartezeit zu erhöhen, während bei höherer Temperatur der Messung sich eine kürzere Wartezeit ergibt, um entsprechende Messergebnisse zu erzielen. Da die Farbänderung in der Reagenzlösung jedoch eine dynamische Farbänderung ist, würde eine zu kurze Zeitdauer zu einem ungenauen Ergebnis führen, während eine zu lange Zeitdauer (bei entsprechend erniedrigter Temperatur) zu einer unnötigen Messdauerverlängerung führt.

Die sich aus dem Eisengehalt der Probe ergebende Färbung in der Reagenzlösung ist sowohl eine Chrominanz- als auch eine Luminanzfärbung. Generell wird die Färbung dunkler, je höher der Eisengehalt ist, während die Farbigkeit (Chrominanz) sich bei kleinem Eisengehalt von einer gelblichen Färbung über eine grünliche Färbung bei höherem Eisengehalt bis zu einer tiefblauen Färbung bei sehr hohem Eisengehalt entwickelt.

In der Fig. 1 ist eine Tabelle dargestellt, die in der ersten Spalte den Gesamteisengehalt in mg/l anzeigt, während in den folgenden Spalten C, M, Y die jeweilig sich ergebenden Cyan-, Magenta- und Yellow-Farbwerte als Prozentangaben dargestellt sind. Diese CMY-Werte sind auf der verwendeten Farbskala bzw. den Farbkarten entsprechend aufgedruckt, so dass daraus beim Vergleich zwischen Reagenzlösung und Farbkarten auf den Eisengehalt im Schmieröl geschlossen werden kann.

Zur weiteren Verbesserung der standardisierten Messbedingungen kann auch vorgesehen sei, die Messung nur bei bestimmten standardisierten Lichtverhältnissen vorzunehmen, z. B. unter Halogen- oder LED-Licht bestimmter Spektraleigenschaften oder einer bestimmten Farbtemperatur.

Bei der Messung korrosiven Eisens besteht die Reagenzlösung vorzugsweise aus 10 ml gereinigtem Wasser mit 0,2 ml 2%iger Kaliumhexacyanoferrat (II) Trihydrat Lösung, welche bei Versuchsbeginn durch Schütteln miteinander vermischt werden. Die Versuchsdurchführung erfolgt entsprechend der Messung des Gesamteisengehalts, wobei jedoch statt 0,5 ml Probe eine Menge von 1 ml verwendet wird und die Ruhezeit statt 8 Minuten 15 Minuten beträgt. Auch in diesem Fall kann durch Änderung der Parameter Temperatur, Zeitdauer, Oberflächengröße im Messbehälter der Verfahrensablauf variiert werden. Er sollte jedoch standardisierten Parametern entsprechen, um eine Vergleichbarkeit der Messergebnisse erreichen zu können.

Fig. 2 zeigt eine grafische Darstellung zur Erläuterung des Zusammenhangs zwischen Messtemperatur und Wartezeit bei der Messung des Gesamteisengehalts, um im Wesentlichen gleiche Ergebnisse der Luminanz- und Chominanz bei einer bestimmten Eisenmenge in einer Probe zu erzielen. Die Grafik zeigt, dass bei erhöhter Temperatur die Wartezeit verringert werden kann, während sie bei niedriger Temperatur beträchtlich zu erhöhen ist. Ein optimaler Wert ergibt sich bei 60°C und 8 Minuten Wartezeit für die Messung der Gesamteisenmenge.

Für die Messung des korrosiven Eisens ergibt sich eine entsprechende Darstellung, wobei der optimale Wartezeitraum jedoch 15 Minuten beträgt. Die verlängerte Reaktionszeit ergibt sich insbesondere daraus, dass bereits oxidiertes Eisen nur in sehr kleinen Mengen im Mikrogrammbereich pro Liter vorkommt und keine Salpetersäure, sondern nur Wasser verwendet wird.

Alternativ zum Einstellen der Reagenzlösung in ein Wasserbad und Abwarten bis die Reagenzlösung die Temperatur des Wasserbads erreicht hat, kann auch vorgesehen sein, die Schmierölprobe zunächst auf die Reagenzlösung aufzugeben und den Messbehälter dann erst in das Wasserbad einzustellen. Dadurch ergibt sich eine geringfügig verlängerte Reaktionszeit, ohne dass sich das Messergebnis verschlechtert.

Der Vergleich der Reagenzlösung mit Farbkarten oder einer Farbskala ist einfach auszuführen und auch ausreichend genau. Es ist aber auch denkbar, die Messung über ein Chrominanzmessgerät durchzuführen, jedoch lässt sich dadurch keine höhere Genauigkeit, soweit sie erforderlich ist, erreichen.

Die verwendete schwache Salpetersäure weist vorzugsweise einen pH-Wert von 2,5 auf und braucht daher nicht als Gefahrgut behandelt werden. Bei dem angegebenen gereinigten Wasser handelt es sich vorzugsweise um destilliertes Wasser.

Die zu verwendenden Chemikalien und Messbehälter werden vorzugsweise in einem Transportkoffer bereitgestellt, der dem Personal an Bord eines Schiffes zur Verfügung gestellt wird. Die Messung kann vom Schiffspersonal selbst durchgeführt werden, ohne dass eine externe Laborauswertung erforderlich ist.

## Patentansprüche

1. Verfahren zur Feststellung des Eisengehalts in einem Schmieröl einer Schiffsantriebsmaschine, welches folgende Schritte umfasst:
- es wird eine Reagenzlösung mit Kaliumhexacyanoferrat (II) bereitgestellt,
- auf die Oberfläche der Reagenzlösung wird eine Probe des Schmieröls zur Bildung einer Messprobe aufgebracht,
- die Messprobe wird für eine Zeitdauer zwischen 4 Minuten und 20 Minuten und bei einer erhöhten Temperatur zwischen 40°C und 80°C ruhig gehalten,
- es wird dann ein Farbvergleich zwischen der Färbung der Reagenzlösung mit einer Farbskala durchgeführt, wobei der Grad der Färbung ein Maß für den Eisengehalt des Schmieröls darstellt.

2. Verfahren nach Anspruch 1, **dadurch gekennzeichnet, dass** zur Messung des Gesamteisengehalts die Reagenzlösung Salpetersäure in einer Konzentration von 0,1 bis 0,5 % und Kaliumhexacyanoferrat (II) in einer Konzentration von 0,002 bis 0,05 % in gereinigtem Wasser umfasst.

3. Verfahren nach Anspruch 2, **dadurch gekennzeichnet, dass** die Reagenzlösung 10 ml einer 0,25%igen Salpetersäure in gereinigtem Wasser und 0,2 ml 2%iges Kaliumhexacyanoferrat (II) in gereinigtem Wasser umfasst.

4. Verfahren nach Anspruch 2 oder 3, **dadurch gekennzeichnet, dass** zur Messung des Gesamteisengehalts eine Probe Schmieröl von 0,5 ml bei einer Reagenzmenge von ca. 10 ml verwendet wird.

5. Verfahren nach Anspruch 4, **dadurch gekennzeichnet, dass** die Ruhezeit 8 Minuten bei einer Temperatur von 60°C beträgt.

6. Verfahren nach Anspruch 1, **dadurch gekennzeichnet, dass** zur Messung des korrosiven Eisengehalts die Reagenzlösung gereinigtes Wasser und Kaliumhexacyanoferrat (II) in einer Konzentration von 0,002 bis 0,05 % umfasst.

7. Verfahren nach Anspruch 6, **dadurch gekennzeichnet, dass** die Reagenzlösung 10 ml gereinigtes Wasser und 0,2 ml 2%iges Kaliumhexacyanoferrat (II) in gereinigtem Wasser umfasst.

8. Verfahren nach Anspruch 7, dadurch gekenntzeichnet, dass zur Messung des korrosiven Eisens eine Probe Schmieröl von 1 ml bei einer Reagenzmenge von ca. 10 ml verwendet wird.

9. Verfahren nach Anspruch 8, **dadurch gekennzeichnet, dass** die Ruhezeit 15 Minuten bei einer Temperatur von 60°C beträgt.

10. Testkit zur Feststellung des Eisengehalts in einem Schmieröls einer Schiffsantriebsmaschine gemäß einem Verfahren nach Anspruch 1, gebildet aus einem Transportkoffer, welcher einen auf eine geregelte Temperatur von 40 - 80°C regelbaren Heizbehälter zur Aufheizung eines oder mehrerer Messbehälter in einem Wasserbad, eine Vorratsflasche einer wässrigen Salpetersäure, eine Vorratsflasche Kaliumhexacyanaferrat (II), eine Vorratsflasche gereinigtes Wasser, eine Reihe von Messbehältern und eine Anzahl von Farbmusterkarten enthält.

11. Testkit nach Anspruch 10, **dadurch gekennzeichnet, dass** es sich bei der Salpetersäure um eine 0,25%ige wässrige Salpetersäure handelt und/oder dass das Kaliumhexacyanoferrat (II) als Kaliumhexacyanoferrat (II) Trihydrat in Pulverform vorliegt und/oder dass die Messbehälter ein Volumen von 17 ml aufweisen.

## Claims

1. Method for determining the iron content in a lubricating oil of a ship's propulsion engine, comprising the following steps:
- preparing a reagent solution that comprises potassium ferrocyanide (II),
- applying a sample of the lubricating oil to the surface of the reagent solution to form a test sample,
- keeping the test sample still for a period of time of between 4 minutes and 20 minutes and at an increased temperature of between 40 °C and 80 °C,
- subsequently making a colour comparison between the colour of the reagent solution and a colour scale, wherein the degree of colour represents a measure for the iron content of the lubricating oil.

2. Method according to claim 1, **characterised in that**, for measuring the total iron content, the reagent solution contains nitric acid in a concentration of from 0.1 to 0.5 % and potassium ferrocyanide (II) in a concentration of from 0.002 to 0.05 % in purified water.

3. Method according to claim 2, **characterised in that** the reagent solution contains 10 ml of a 0.25 % nitric acid in purified water and 0.2 ml 2 % potassium ferrocyanide (II) in purified water.

4. Method according to either claim 2 or claim 3, **characterised in that**, for measuring the total iron content, a 0.5 ml sample of lubricating oil is used for a reagent quantity of approximately 10 ml.

5. Method according to claim 4, **characterised in that** the resting time is 8 minutes at a temperature of 60 °C.

6. Method according to claim 1, **characterised in that**, for measuring the corrosive iron content, the reagent solution contains purified water and potassium ferrocyanide (II) in a concentration of from 0.002 to 0.05 %.

7. Method according to claim 6, **characterised in that** the reagent solution contains 10 ml purified water and 0.2 ml 2 % potassium ferrocyanide (II) in purified water.

8. Method according to claim 7, **characterised in that**, for measuring the corrosive iron, a 1 ml sample of lubricating oil is used for a reagent quantity of approximately 10 ml.

9. Method according to claim 8, **characterised in that** the resting time is 15 minutes at a temperature of 60 °C.

10. Assay kit for determining the iron content in a lubricating oil of a ship's propulsion engine according to a method according to claim 1, formed of a carry case that contains a heating vessel, which can be controlled to a controlled temperature of 40 to 80 °C for heating one or more measuring vessels in a water bath, a reservoir bottle of an aqueous nitric acid, a reservoir bottle of potassium ferrocyanide (II), a reservoir bottle of purified water, an array of measuring vessels and a number of colour control charts.

11. Assay kit according to claim 10, **characterised in that** the nitric acid is a 0.25 % aqueous nitric acid and/or **in that** the potassium ferrocyanide (II) is present in powder form as potassium ferrocyanide (II) trihydrate and/or **in that** the measuring vessels have a volume of 17 ml.

## Revendications

1. Procédé pour déterminer la teneur fer dans une huile de lubrification d'un moteur de bateau, qui comprend les étapes suivantes :
- une solution de réactif avec de l'hexacyanoferrate (II) de potassium est préparée,
- sur la surface de la solution de réactif est appliqué un échantillon d'huile de lubrification, pour former un échantillon de mesure,
- on laisse reposer l'échantillon de mesure pendant une durée située entre 4 minutes et 20 minutes et à une température élevée située entre 40°C et 80°C,
- une comparaison de couleurs est ensuite réalisée ente la coloration de la solution de réactif et une échelle de couleurs, le degré de coloration représentant une mesure pour la teneur en fer de l'huile de lubrification.

2. Procédé selon la revendication 1, **caractérisé en ce que** pour la mesure de la teneur totale en fer, la solution de réactif comprend de l'acide nitrique dans une concentration de 0,1 à 0,5 % et de l'hexacyanoferrate (II) de potassium dans une concentration de 0,002 à 0,05 % dans de l'eau purifiée.

3. Procédé selon la revendication 2, **caractérisé en ce que** la solution de réactif comprend 10 ml d'un acide nitrique à 0,25 % dans de l'eau purifiée, et 0,2 ml d'hexacyanoferrate (II) de potassium à 2 % dans de l'eau purifiée.

4. Procédé selon la revendication 2 ou 3, **caractérisé en ce que** pour la mesure de la teneur totale en fer, on utilise un échantillon d'huile de lubrification de 0,5 ml pour une quantité de réactif d'environ 10 ml.

5. Procédé selon la revendication 4, **caractérisé en ce que** la durée de repos est de 8 minutes à une température de 60°C.

6. Procédé selon la revendication 1, **caractérisé en ce que** pour la mesure de la teneur en fer corrosif, la solution de réactif comprend de l'eau purifiée et de l'hexacyanoferrate (II) de potassium dans une concentration de 0,002 à 0,05 %.

7. Procédé selon la revendication 6, **caractérisé en ce que** la solution de réactif comprend 10 ml d'eau purifiée et 0,2 ml d'hexacyanoferrate (II) de potassium à 2 % dans de l'eau purifiée.

8. Procédé selon la revendication 7, **caractérisé en ce que** pour la mesure du fer corrosif, on utilise un échantillon d'huile de lubrification de 1 ml pour une quantité de réactif d'environ 10 ml.

9. Procédé selon la revendication 8, **caractérisé en ce que** la durée de repos est de 15 minutes à une température de 60°C.

10. Kit de test pour déterminer la teneur en fer dans une huile de lubrification d'un moteur de bateau avec un procédé selon la revendication 1, composé d'un coffre de transport qui contient un récipient de chauffage, réglable à une température régulée de 40-80°C, pour chauffer un ou plusieurs récipients de mesure dans un bain-marie, une bouteille de stockage d'acide nitrique aqueux, une bouteille de stockage d'hexacyanoferrate (II) de potassium, une bouteille de stockage d'eau purifiée, une série de récipients de mesure et un certain nombre de cartes d'échantillons de couleurs.

11. Kit de test selon la revendication 10, **caractérisé en ce qu'**il s'agit, pour l'acide nitrique, d'un acide nitrique aqueux à 0,25 % et/ou **en ce que** l'hexacyanoferrate (II) de potassium se présente comme un hexacyanoferrate (II) de potassium trihydraté sous forme de poudre et/ou **en ce que** les récipients de mesure présentent un volume de 17 ml.
